# EUROPEAN PATENT SPECIFICATION

(11) **EP 1 691 879 B1**
(45) Date of publication and mention of the grant of the patent: **03.06.2009**
(21) Application number: 04811751.9
(22) Date of filing: 19.11.2004
(51) Int. Cl.: A61M 29/00

(54) **DEVICE FOR CAVITY OBLITERATION**
VORRICHTUNG FÜR DIE OBLITERATION EINES HOHLRAUMS
DISPOSITIF D'OBLITERATION D'UNE CAVITE

(30) Priority: 20.11.2003 US 524366 P
(43) Date of publication of application: 23.08.2006
(73) Proprietor: The Catheter Exchange, Inc., Encino, California 91436 (US)
(72) Inventor: DEUTSCH, Harvey L., Los Angeles, California 90024 (US)
(74) Representative: Garavelli, Paolo
(86) International application number: PCT/US2004/039089
(87) International publication number: WO 2005/051172

(56) References cited:
- EP-B1- 0 664 104
- WO-A-97/15315
- WO-A2-01/97740
- US-A- 5 041 090
- US-A- 5 049 132
- US-A- 5 588 961
- US-B1- 6 379 329

## Description

### BACKGROUND

There are a variety of diseases and conditions in humans and in animals which result in the creation of abnormal cavities which cause real or potential morbidity *in vivo.* For example, patients with advanced emphysema typically have chronic bullous formations within the superior lung tissue that can render the patient symptomatic due to pressure from the formation. Other examples of abnormal cavities include enteric or urinary fistulas, large varicosities, and arteriovenous fistulas. Similarly, iatrogenic cavities are created by large surgical incisions used in major open abdominal and spinal surgeries. Closure of such large surgical incisions requires repair generally performed in multiple layers, sometimes using metal wires to secure the closure. Large surgical incision closures pose the risk of wound dehiscence, as well as abdominal wall hernias, hematomas, abscesses, lymphoceles, and seromas.

One of the most common abnormal cavities created by a disease or condition in humans which requires medical intervention is an inguinal hernia, either direct or indirect. Inguinal hernias are treated in a variety of ways, for example, such as by utilizing an external truss. Large inguinal hernias or inguinal hernias containing or potentially containing small bowel generally require surgical obliteration. Generally, surgical obliteration for inguinal hernias involves an open procedure comprising incising the integument and abdominal wall overlying the hernia sac, opening the hernia sac, and obliterating the hernia sac using sutures, with or without placing a mesh to reinforce the abdominal wall and prevent recurrences.

More recently, endoscopic procedures for the repair of inguinal hernias have been developed which are less invasive than open procedures, however, both open procedures and endoscopic procedures have a significant recurrence rate estimated at between 1 and 10 percent depending on the study. Further, open procedures in particular have a significant recovery time associated with the repair. Additionally, both open procedures and endoscopic procedures are relatively expensive.

US-B1-6 379 329 discloses a detachable balloon embolization device and method according to the preamble of Claim 1. US-A-5 041 090 discloses an occluding device.

Therefore, it would be useful to have a new method for the obliteration of abnormal cavities *in vivo* caused by diseases or conditions, where the cavities cause real or potential morbidity. Preferably, the new method would also be less traumatic and less expensive than present method. Further, the new method would be rapid, and would be useful in patients with significant underlying diseases which place them at risk for more invasive surgical procedures. Additionally, the new method would not be associated with long recoveries.

### SUMMARY

According to the present invention, there is provided a device according to claim 1.

### FIGURES

These and other features, aspects and advantages of the present invention will become better understood from the following description, appended claims, and accompanying figures where:
Figure 1 is a partial lateral perspective view of one embodiment of a device according to the present invention;
Figure 2 is an enlarged, cutaway, lateral perspective view of the distal end of the device shown in Figure 1;
Figure 3 is an enlarged, cross-sectional, perspective view of the device shown in Figure 1 taken along the line 3-3;
Figure 4 is an enlarged, cross-sectional, perspective view of the device shown in Figure 1 taken along the line 4-4;
Figure 5 is a partial, lateral prospective view of the distal segment of another embodiment of the device according to the present invention;
Figure 6 is a partial, lateral prospective view of the distal segment of another embodiment of the device according to the present invention;
Figure 7 through Figure 11 are cross-sectional, perspective views of some of the steps of one embodiment of the method according to the present invention for obliterating a cavity;
Figure 12 through Figure 19 are cross-sectional, perspective views of some of the steps of one embodiment of a method according to the present invention for obliterating the cavity of an inguinal hernia;
Figure 20 is an anchor useful in a method according to the present invention;
Figure 21 and Figure 22 are lateral perspective views of the distal segment of an alternate embodiment of a device useful in the obliteration of cavities caused by a disease or condition which causes real or potential morbidity, shown in the expanded and collapsed configuration;
Figure 23 is a lateral perspective view of another embodiment of the device according to the present invention;
Figure 24 is a cross-sectional, perspective view of the device shown in Figure 23, taken along the line 24-24; and
Figure 25 is a lateral, cutaway perspective view of the device shown in Figure 23.

### DESCRIPTION

According to one embodiment of the present invention, there is provided a device that can be used to obliterate a cavity caused by a disease or condition, where the cavity causes real or potential morbidity. According to an example useful for understanding the invention, there is provided a method for obliterating a cavity caused by a disease or condition, where the cavity causes real or potential morbidity. In one example, the method comprises providing a device according to the present invention. The method of the example is relatively less traumatic and relatively less expensive than open procedures, and is rapid and useful in patients with significant underlying diseases which place them at risk for more invasive surgical procedures, and is not associated with long recoveries. The device and method will now be disclosed in greater detail.

As used herein, the term "comprise" and variations of the term, such as "comprises" and "comprising," are not intended to exclude other additives, components, integers or steps.

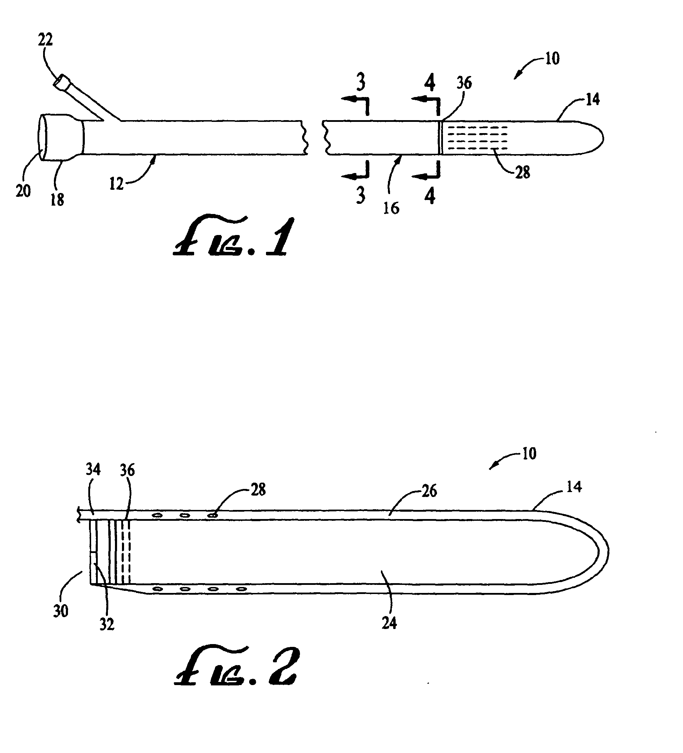

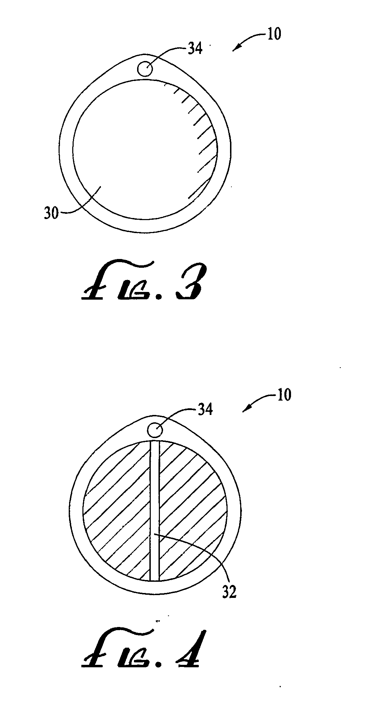

According to one embodiment of the present invention, there is provided a device that can be used to obliterate a cavity caused by a disease or condition, where the cavity causes real or potential morbidity. Referring now to Figure 1 to Figure 4 there are shown, respectively, a partial lateral perspective view of one embodiment of the device according to the present invention (Figure 1); an enlarged, cutaway, lateral perspective view of the distal end of the device shown in Figure 1 (Figure 2); an enlarged, cross-sectional, perspective view of the device shown in Figure 1 taken along the line 3-3 (Figure 3); and an enlarged, cross-sectional, perspective view of the device shown in Figure 1 taken along the line 4-4 (Figure 4). As can be seen, the device 10 generally comprises a proximal segment 12, a distal segment 14, and an intermediate segment 16 between the proximal segment 12 and distal segment 14. Preferably, the proximal end of the proximal segment 12 comprises at least one connector 18 comprising a balloon layer inflation and deflation port 20, and comprises an adhesive delivery port 22. In one embodiment, as shown in Figure 1, the inflation and deflation port 20 is separate from the adhesive delivery port 22. Alternately, however, the inflation and deflation port 20 and the adhesive delivery port 22 can be combined into a single port, not shown, as will be understood by those with skill in the art with reference to this disclosure.

In one embodiment, as shown in Figure 1 and Figure 2, the distal segment 14 of the device 10 comprises an inner balloon layer 24 surrounded by an outer balloon layer 26. The outer balloon layer 26 surrounding the inner balloon layer 24 comprises a plurality of perforations 28. In a preferred embodiment, all of the perforations 28 in the outer balloon layer 26 are in the proximal portion of the outer balloon layer 26 adjacent the distal end of the intermediate segment 16, though other arrangements of the perforations 28 can be used, as will be understood by those with skill in the art with reference to this disclosure.

The device 10 further comprises at least two lumens. One lumen, an inflation and deflation lumen 30, connects the inflation and deflation port 20 in the proximal segment 12 with the inner balloon layer 24 in the distal segment 14 through a self-sealing valve 32. Another lumen, an adhesive delivery lumen 34, connects the adhesive delivery port 22 in the proximal segment 12 with the outer balloon layer 26 in the distal segment 14. In one embodiment, as shown in Figure 2, the device 10 further comprises a separation area 36 between the distal segment 14 and the intermediate segment 16 configured to separate the distal segment 14 from the intermediate segment 16, when the device 10 is separated along the separation area.

The distal segment 14 at least, and preferably, the entire device 10, comprises biocompatible material. The device is made according to techniques known to those with skill in the art, as will be understood by those with skill in the art with reference to this disclosure.

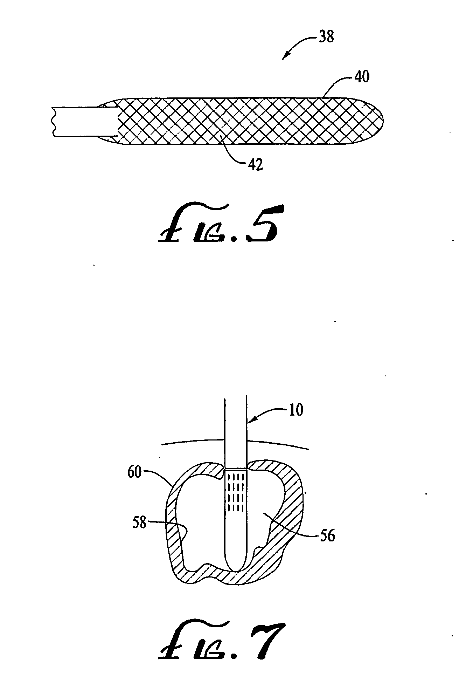

Referring now to Figure 5, there is shown a partial, lateral prospective view of the distal segment of another embodiment of the device according to the present invention. As can be seen, in this embodiment, the device 38 further comprises a fine mesh layer 40 completely surrounding the outer balloon layer 42. The fine mesh layer 40 can be any suitable biocompatible material. In a preferred embodiment, the mesh comprises one or more substance selected from the group consisting of polypropylene, polyethylene, polytetrafluoroethylene and polyglycolic acid. Any suitable biocompatible substance can be used, however, as will be understood by those with skill in the art with reference to this disclosure.

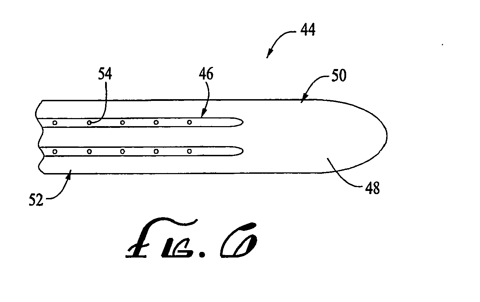

Referring now to Figure 6, there is shown a partial, lateral prospective view of the distal segment of another embodiment of the device according to the present invention. As can be seen, in this embodiment, the device 44 comprises one or more than one microcatheter 46 attached to the external surface of the inner balloon layer 48 of the distal segment 50 in place of the outer balloon layer 26 of the devices 10 and 38 shown in Figure 1 through Figure 4. Preferably, each microcatheter 46 is in communication with the adhesive delivery port, not shown, through a single adhesive delivery lumen in the intermediate segment 52. As will be understood by those with skill in the art with reference to this disclosure, however, a plurality of microcatheters could also be connected to the adhesive delivery port by a plurality of adhesive delivery lumens. Further, preferably, each of the one or more than one microcatheter 46 comprises a plurality of perforations 54 to the perforations 28 in the outer balloon layer 26 in the embodiment of the device 10 shown in Figure 1 and Figure 2. The one or more than one microcatheter 46 can extend from the proximal end of the distal segment 50 to the distal end of the distal segment 50, or can extend only partway toward the distal end of the distal segment 50, as shown in Figure 6. Preferably, however, each microcatheter preferably has a plurality of perforations 54 only in the proximal portion of the microcatheter 46 at the distal segment 50. In a preferred embodiment, the device 44 comprises between three and six microcatheters 46. In another preferred embodiment, each microcatheter 46 has an inner diameter less than about 2 French.

According to an example, there is provided a method for obliterating a cavity caused by a disease or condition, where the cavity causes real or potential morbidity. The method comprises, first, selecting a patient with a cavity requiring obliteration. In one example, the cavity is selected from the group consisting of a chronic bullous formation, an enteric fistula, a urinary fistula, a varicosity and an arteriovenous fistula, an inguinal hernia sac and the stomach, though a large variety of cavities are susceptible to obliteration by the present method, as will be understood by those with skill in the art with reference to this disclosure. In a preferred example, the condition is severe obesity and the cavity is formed by the gastric mucosa, where the method is used to decrease the potential volume of the stomach as part of a treatment for severe obesity.

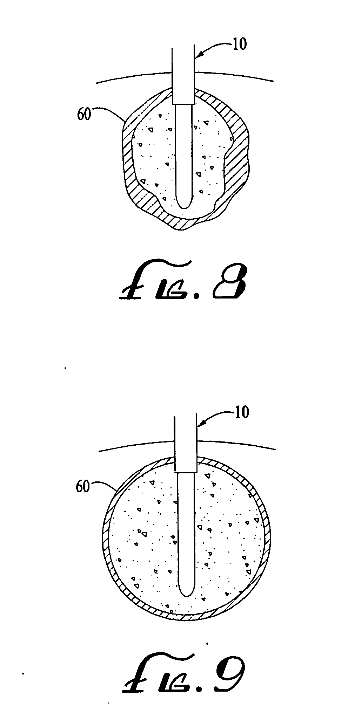

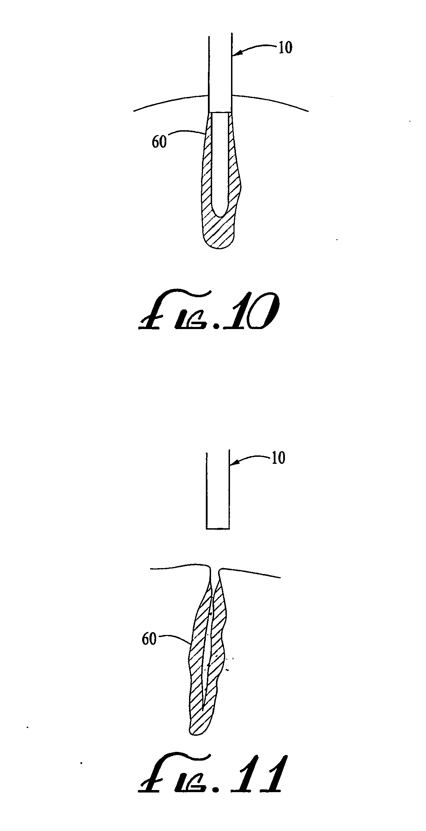

Referring now to Figure 7 through Figure 11, there are shown cross-sectional, perspective views of some additional steps of one example of a method for obliterating such a cavity.

As can be seen, after selecting the patient, a device for obliteration of the cavity is provided. In a preferred embodiment, the device is a device according to one embodiment of the present invention, such as the device 10 shown in the Figure 1 through Figure 4, though other devices, such as another device according to the present invention can be used. Next, the device 10 is inserted into the cavity 56 until the distal segment 14 of the device 10 lies within the cavity 56. The inner balloon layer 24 is then inflated causing both the inner balloon layer 24 and outer balloon layer 26, or any layer or structures external to the inner balloon layer 24, to contact the inner surface 58 of the wall 60 of the cavity 56. Then, an adhesive is introduced through the adhesive delivery lumen external to the inner balloon layer 24, such as into the potential space between the inner balloon layer 24 and the outer balloon layer 26, or other layer or structures external to the inner balloon layer, causing adhesive to extrude through the perforations 28 in the outer balloon layer 26 or corresponding structures, and spread between the device 10 and the inner surface 58 of the wall 60 of the cavity 56, thereby binding the outer balloon layer 26 to the inner surface 58 of the wall 60 of the cavity 56. Next, the inner balloon layer 24 is deflated through the inflation and deflation lumen, thereby contracting the previously expanded wall 60 of the cavity 56 until the cavity 56 surrounds the deflated distal segment 14 of the device 10, thereby obliterating the cavity 56. Then, the distal segment 14 of the device 10 is detached at the separation area 36 leaving the distal segment 14 of the device 10 within the obliterated cavity 56 and surrounded by the obliterated cavity 56, while the proximal segment 12 and intermediate segment 16 of the device 10 are removed.

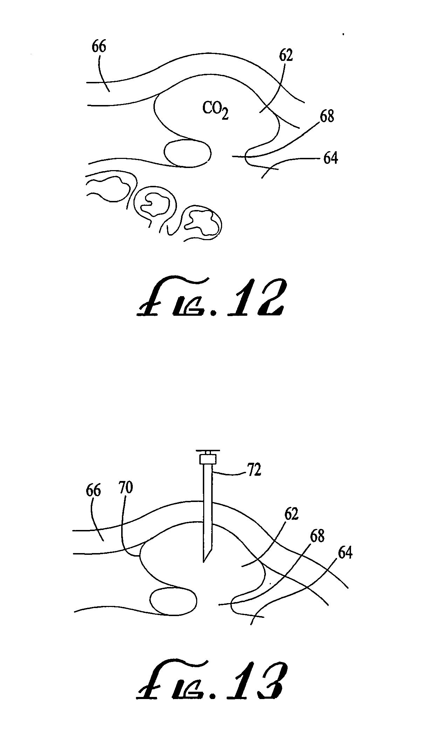

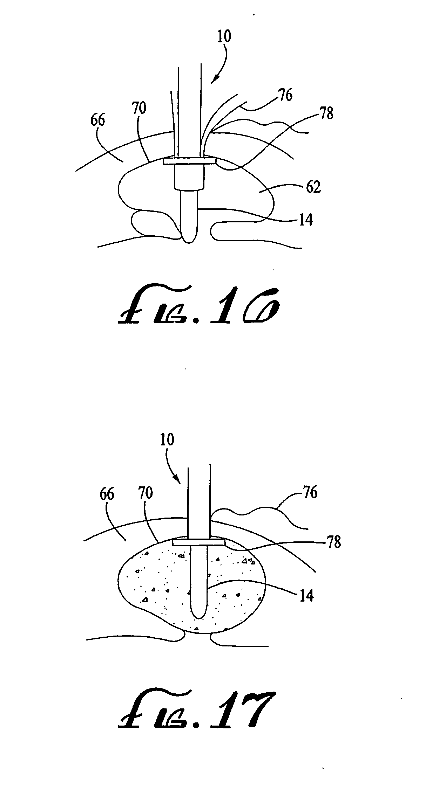

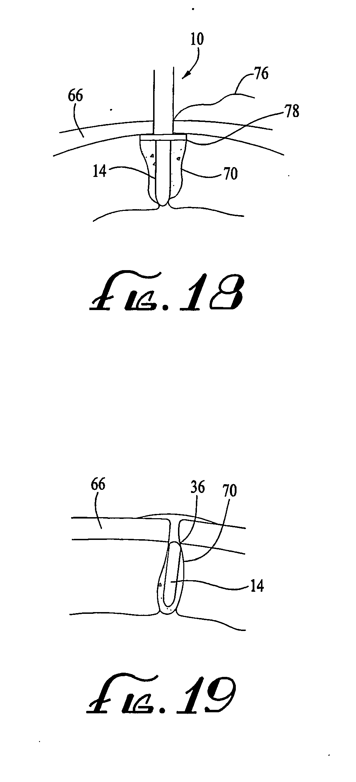

By way of example only, the method will now be disclosed with respect to obliterating the cavity of an inguinal hernia in a patient, that is, a hernia sac. However, corresponding steps can be used to obliterate other cavities, as will be understood by those with skill in the art with reference to this disclosure. Referring now to Figure 12 through Figure 19, there is shown cross-sectional, perspective views of some of the steps of one example of a method for obliterating the cavity of an inguinal hernia.

The method comprises, first, selecting a patient with an inguinal hernia with a hernia sac 62 suitable for obliteration according to the present method. After selecting the patient, the remaining steps of the method are performed using an imaging technique as required, such as a technique selected from the group consisting of thin cut computerized tomography, fluoroscopy, rapid magnetic resonance imaging, digital rotational angiography with three-dimensional reconstruction, ultrasound, and another suitable technique, and a combination of the preceding. The patient is anesthetized as required, such as by I.V. sedation and local skin anesthesia. Next, the peritoneal cavity 64 is accessed through a small opening made through the integument and abdominal wall 66. In a preferred embodiment, the opening is made using a small needle, such as a 0.5309 mm to 0.8357 mm needle. Further, preferably, the opening is made in the periumbilical region. In a preferred embodiment, once the peritoneal cavity 64 is accessed, a biocompatible gas, such as a carbon dioxide, is introduced through the opening into the peritoneal cavity 64 causing the peritoneal cavity 64 and hernia sac 62 to distend, as shown in Figure 12, according to techniques well known to those with skill in the art. Additionally, in a preferred example, the patient pelvis is elevated relative to the patient's abdomen to encourage the biocompatible gas to enter the hernia sac 62 through the proximal communication 68 between the hernia sac 62 and the peritoneal cavity 64.

Next, an appropriate site for creating an opening into the hernia sac 62 is located using an appropriate imaging technique. Then, an opening is made through the integument and abdominal wall 66 and hernia sac wall 70 into the hernia sac 62. In a preferred embodiment, the opening is made using a needle 72, such as an 0.8357 mm to 1.2141 mm needle, as shown.

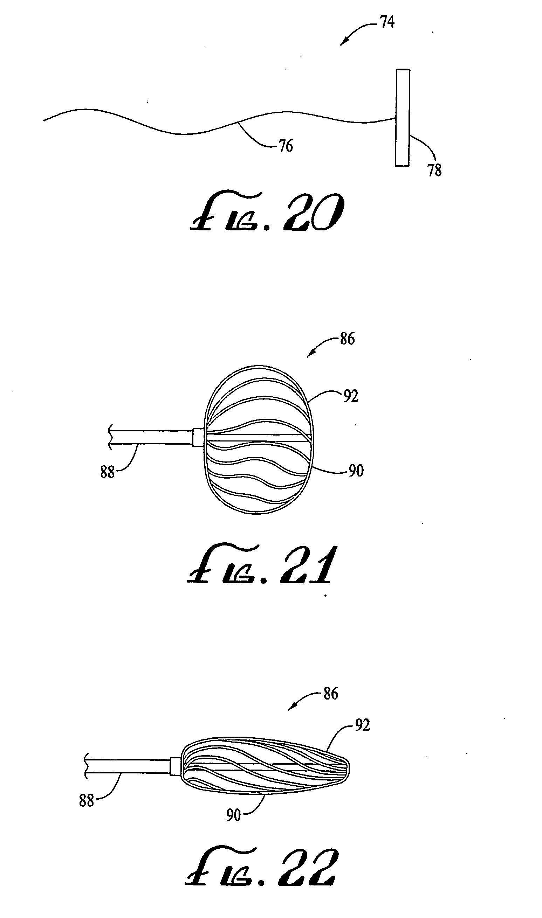

Next, in a preferred example, an anchor 74 is introduced through the needle 72. Referring now to Figure 20, there is shown an anchor 74 useful in the present method. As can be seen, the anchor 74 comprises a proximal portion 76 attached to a distal portion 78. The proximal portion 76 comprises an elongated thread-like structure, such as for example a biocompatible suture material such as VICRYL^{®}. The distal portion 78 comprises a relatively stiff elongated structure connected to the proximal portion 76 at the approximate center of the long axis of the distal portion 78. The distal portion 78 can comprise any suitable biocompatible material, as will be understood by those with skill in the art with reference to this disclosure. In a preferred example, the distal portion 78 comprises polyglycolic acid. In another preferred example, the distal portion 78 comprises guidewire material comprising a length between about 8 mm to about 10 mm.

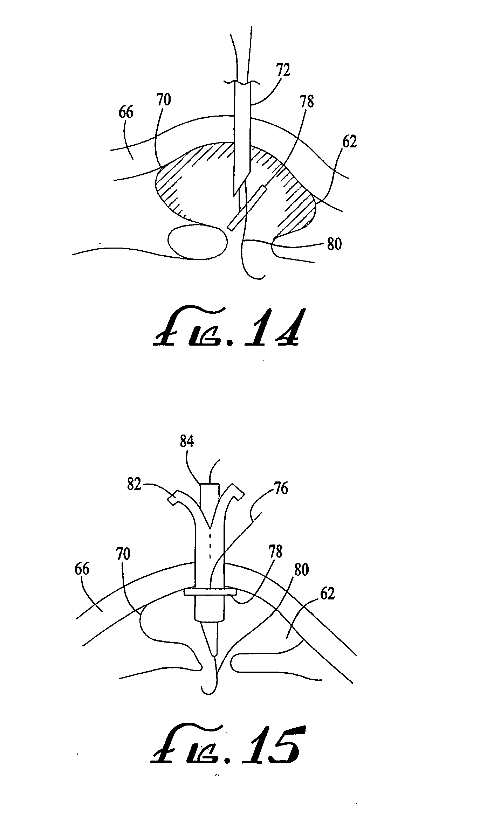

As shown in Figure 14, the distal portion 78 of the anchor 74 and a guidewire 80 are advanced through the lumen of the needle 72 until the distal portion 78 extends completely through the distal end of the needle 72 and into hernia sac 62. The needle 72 is then removed from the hernia sac 62 and overlying structures leaving the anchor 74 and guidewire 80 in place.

Next, the proximal portion 76 of the anchor 74, if used, is retracted proximally approximating the wall of the hernia sac 62 with the integument and abdominal wall 66. Then, a peel-away sheath 82 with a central dilator 84, such as a peel-away sheath, is inserted over the guidewire 80, and the distal end of the peel-away sheath 82 and dilator 84 are advanced into the hernia sac 62. Then, the dilator 84 and guidewire 80 are removed.

Next, a device for obliterating a cavity, such as a device 10 according to the present invention, is inserted through the peel-away sheath 82 until the distal segment 14 of the device 10 is completely within the hernia sac 62. Then, the peel-away sheath 82 is removed from the hernia sac 62 completely.

Next, the inner balloon layer is inflated through the inflation and deflation lumen using a suitable inflation material, until the surface of the distal segment substantially contacts the wall of the hernia sac 62. The inflation material can be, for example, air, saline, or a gas such as carbon dioxide. Proximal traction on the anchor, if used, is used to assist in this maneuver. After inflation, correct positioning of the device is verified using an imaging technique.

Next, an adhesive is then delivered through the adhesive delivery port of the device 10 into the space between the outer surface of the inner balloon layer 24 and the inner surface of the hernia sac wall 70. Any suitable biocompatible adhesive can be used, such as for example, a cyanoacrylate such as N-butyl cyanoacrylate (NBCA), or DERMABOUND^{®} (Johnson & Johnson Corp., New Brunswick, NJ US). Preferably, the adhesive requires an ionic environment to become activated and cured, so that it will not cure within the balloon, but only when in the ionic environment of the cavity. The adhesive is left to cure until adhesion has been achieved between the distal segment 14 of the device 10 and the hernia sac wall 70.

Then, the inner balloon 24 of the distal segment 14 of the device is deflated by withdrawing the inflation material from the inflation and deflation port. This deflation causes the hernia sac wall 70 to implode, thereby obliterating the cavity of the hernia sac 62.

Next, the distal segment 14 of the device 10 is detached at the separation area 36 from the proximal segment 12 and intermediate segment 16 of the device 10. Finally, the opening through the integument and abdominal wall 66 into the hernia sac 62 is closed in a routine manner as will be understood by those with skill in the art with reference to this disclosure, such as by sutures, staples, and routine post-procedure care would be instituted. If necessary, post-procedure imaging can be performed to confirm obliteration of the cavity.

The method can be performed using any device according to the present invention as is suitable for the cavity. Additionally, other devices could also be used if appropriate. For example, referring now to Figure 21 and Figure 22, there is shown a lateral perspective view of the distal segment of an alternate embodiment of a device 86 useful in the obliteration of cavities caused by real or potential morbidity. As can be seen, the device 86 comprises a proximal segment 88 and a distal segment 90. The distal segment 88 of the device 86 comprises a plurality of axially arranged wire-like structures 92 forming a basket, such as is used for percutaneous retrieval of urinary and biliary calculi, and for intravascular foreign body retrieval. During insertion, the distal segment 88 is collapsible down to a low profile as shown in Figure 22. Once deployed within the target cavity, the distal segment 88 of the device 86 is expanded to approximate the cavity wall, and an adhesive is introduced through thin sleeves that allow the adhesive to express out along the length of the basket wires. This causes adherence of the wires to the cavity wall, and obliteration of the cavity upon collapsing the distal segment 88.

According to another example, the device according to the present invention includes a removable guidewire in the inner balloon layer to assist in locating the device within the cavity to be obliterated, such as for example, within a long varicose vein.

According to another example, the method for obliterating a cavity according to the present invention comprises providing an introducing catheter comprising a lumen with a sealing wire within the lumen. After the cavity is obliterated using a device according to the present invention, the sealing wire is retracted causing the end to loop tightly around the distal segment of the device, thereby sealing off the proximal end of the inflation and deflation lumen. Then, the sealing wire is cut and the introduction catheter is removed.

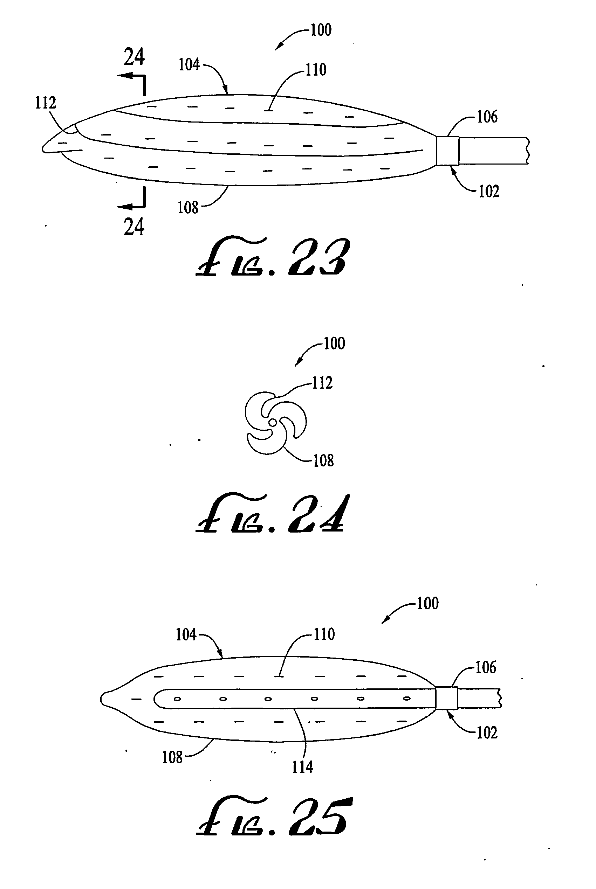

According to another example, there is provided a device that can be used to obliterate a cavity caused by a disease or condition, where the cavity causes real or potential morbidity. Referring now to Figure 23 to Figure 25, there are shown, respectively, a lateral perspective view of one example of the device (Figure 23); a cross-sectional, perspective view of the device shown in Figure 23, taken along the line 24-24 (Figure 24); and a lateral, cutaway perspective view of the device shown in Figure 23 (Figure 25). As can be seen, the device 100 comprises a proximal segment 102 and a distal segment 104. In one example, as shown in Figure 23, the proximal segment 102 comprises a self-sealing valve 106. The distal segment comprises an inflatable balloon 108. The balloon 108 comprises a plurality of perforations 110. In a preferred example, the perforations 110 are distributed throughout the balloon 108 as shown, though other arrangements of the perforations 110 can be used, as will be understood by those with skill in the art with reference to this disclosure. In a preferred example, the balloon is folded into pleats 112 in the uninflated state, as shown in Figure 23 and Figure 24, to permit placement of the device 100 through narrow openings. The device 100 can further comprise a delivery system and detachment system, such as the adhesive delivery and aspiration catheter 114 shown in Figure 25.

According to another example , there is provided a method for obliterating a cavity caused by a disease or condition, where the cavity causes real or potential morbidity. The method comprises, first, selecting a patient with a cavity requiting obliteration, as previously disclosed in this disclosure. Next, a device for obliteration of the cavity is provided, such as the device 100. Then, the device 100 is placed on an adhesive delivery and aspiration catheter 114, as shown in Figure 23 and Figure 25, inserted into the cavity until the device 100 lies within the cavity. Next, the balloon 108 is inflated as shown in Figure 25 by introducing adhesive, or adhesive combined with a biocompatible diluting liquid such as a dextrose solution, through the catheter 114 into the balloon 108 until the inflated balloon 108 generally contacts the wall of the cavity. Adhesive, as disclosed in this disclosure, is then allowed to exit the balloon 108 through the perforations 110, thereby adhering the balloon 108 to the wall of the cavity. The adhesive remaining in the balloon is then aspirated through the catheter 114 causing the balloon 108 to deflate, contracting the wall of the cavity and, thereby, obliterating the cavity. Then, the catheter 114 is removed leaving the device 100 within the cavity. In another example, the device 100 is detached from the catheter 114 using a sealing wire as disclosed in this disclosure. In this example, the delivery system and detachment system comprises an introducing catheter comprising a lumen with a sealing wire within the lumen, and the method comprises retracting the sealing wire causing the end to loop tightly around the proximal segment of the device, thereby sealing off the balloon. Other detachment systems can also be used, as will be understood by those with skill in the art with reference to this disclosure.

Although the present invention has been discussed in considerable detail with reference to certain preferred embodiments, other embodiments are possible. Therefore, the scope of the appended claims should not be limited to the description of preferred embodiments contained in this disclosure.

## Claims

1. Device (44) that can be used to obliterate a cavity comprising:
a) a proximal segment (12) comprising a proximal end;
b) a distal segment (50) comprising an inner balloon layer (48);
c) an intermediate segment (52) between the proximal segment (12) and the distal segment (50);
d) a connector (18) on the proximal end of the proximal segment (12), the connector (18) comprising a balloon layer inflation and deflation port (20) and further comprising an adhesive delivery port (22);
e) an inflation and deflation lumen (30) connecting the inflation and deflation port (20) with the inner balloon layer (48); and
f) an adhesive delivery lumen (34);
g) a self-sealing valve (32) connected to the inflation and deflation lumen (30); and
h) a separation area (36) between the distal segment (50) and the intermediate segment (52) configured to separate the distal segment (50) from the intermediate segment (52);
**characterised in that:**
- said inner balloon layer (48) has more than one microcatheter (46) attached to the external surface of the inner balloon layer (48), each microcatheter (46) comprising a plurality of perforations (54);
- said adhesive delivery lumen (34) connects the adhesive delivery port (22) with the more than one microcatheter (46).

2. Device according to claim 1, where the inflation and deflation port (20) and the adhesive delivery port (22) are combined in a single port.

## Patentansprüche

1. Vorrichtung (44), die für das Schließen eines Hohlraums verwendet werden kann und folgendes einschließt:
a) ein nahe liegendes Segment (12), das ein nahe liegendes Segment enthält;
b) ein distales Segment (50), das intern eine Ballonschicht enthält (48);
c) ein Zwischensegment (52) zwischen dem nahen Segment (12) und dem distalen Segment (50);
d) einen Verbinder (18) am nahe liegenden Endstück des nahe liegenden Segments (12), der Verbinder (18) enthält eine Öffnung zum Aufblasen und Luftablassen der Ballonschicht (20) und enthält außerdem eine Öffnung für die Ausgabe von Adhäsiven (22);
e) ein Lumen zum Aufblasen und Luftablassen (30), das die Öffnung zum Aufblasen und Luftablassen (20) mit der internen Ballonschicht (48) verbindet; und
f) ein Lumen zur Ausgabe von Adhäsiven (34);
g) ein selbstdichtendes Ventil (32), das mit dem Lumen zum Aufblasen und Luftablassen (30) verbunden wird; und
h) einen Trennungsbereich (36) zwischen dem distalen Segment (50) und dem Zwischensegment (52), der so konfiguriert ist, dass er das distale Segment (50) vom Zwischensegment (52) trennt;
und **dadurch gekennzeichnet ist, dass**:
- die genannte interne Ballonschicht (48) mehr als einen Mikrokatheter (46) hat, der an der Außenfläche der internen Ballonschicht (48) befestigt ist, jeder Mikrokatheter (46) enthält mehrere Perforationen (54);
- das genannte Lumen für die Ausgabe von Adhäsiven (34) die Öffnung für die Ausgabe von Adhäsiven (22) mit mehreren Mikrokathetern (46) verbindet.

2. Vorrichtung gemäß Patentanspruch 1, wo die Öffnung zum Aufblasen und Luftablassen (20) und die Öffnung für die Ausgabe von Adhäsiven (22) in einer einzelnen Öffnung zusammengefügt wurden.

## Revendications

1. Dispositif (44) pouvant être utilisé pour obstruer une cavité comprenant:
a) un segment proche (12) comprenant une extrémité proche;
b) un segment distal (50) comprenant une couche de baudruche interne (48);
c) un segment intermédiaire (52) entre le segment proche (12) et le segment distal (50);
d) un connecteur (18) à l'extrémité proche du segment proche (12), ledit connecteur (18) comprenant une ouverture de gonflage et de dégonflage de la couche de baudruche (20) et comprenant encore une ouverture de fourniture d'adhésif (22);
e) un lumen de gonflage et de dégonflage (30) qui relie l'ouverture de gonflage et de dégonflage (20) à la couche de baudruche interne (48); et
f) un lumen de fourniture d'adhésif (34);
g) un clapet hermétique (32) relié au lumen de gonflage et de dégonflage (30); et
h) un espace de séparation (36) entre le segment distal (50) et le segment intermédiaire (52) configuré de manière à séparer le segment distal (50) du segment intermédiaire (52);
**caractérisé en ce que**:
- ladite couche de baudruche interne (48) possède plus d'un micro cathéter (46) attaché à la surface extérieure de la couche de baudruche interne (48), chacun des micro cathéters (46) comprenant une pluralité de perforations (54);
- ledit lumen de fourniture d'adhésif (34) relie l'ouverture de fourniture d'adhésif (22) à plus d'un micro cathéter (46).

2. Dispositif selon la revendication 1, où l'ouverture de gonflage et de dégonflage (20) et l'ouverture de fourniture d'adhésif (22) sont réunies en une seule et unique ouverture.
